# EUROPÄISCHE PATENTSCHRIFT

(11) **EP 1 838 230 B1**
(45) Veröffentlichungstag und Bekanntmachung des Hinweises auf die Patenterteilung: **05.03.2008**
(21) Anmeldenummer: 05700318.8
(22) Anmeldetag: 18.01.2005
(51) Int. Cl.: A61B 17/70

(54) **WINKELSTABILE VORRICHTUNG ZUR GEGENSEITIGEN FIXIERUNG EINES LONGITUDINALEN LÄNGSTRÄGERS MIT EINEM KNOCHENFIXATIONSELEMENT**
ANGULARLY STABLE DEVICE FOR MUTUALLY FIXING A LONGITUDINAL CARRIER TO A BONE FIXING ELEMENT
DISPOSITIF A STABILITE ANGULAIRE POUR FIXATION MUTUELLE D'UN SUPPORT LONGITUDINAL A UN ELEMENT DE FIXATION D'OS

(43) Veröffentlichungstag der Anmeldung: 03.10.2007
(73) Patentinhaber: Synthes GmbH, 4436 Oberdorf (CH)
(72) Erfinder: MATTHYS, Romano, CH-7235 Fideris (CH)
(74) Vertreter: Lusuardi, Werther
(86) Internationale Anmeldenummer: PCT/CH2005/000023
(87) Internationale Veröffentlichungsnummer: WO 2006/076815

(56) Entgegenhaltungen:
- WO-A-20/04021901
- DE-A1- 19 534 136
- DE-U1-7202004 018 03

## Beschreibung

Die Erfindung bezieht sich auf eine Vorrichtung gemäss dem Oberbegriff des Patentanspruchs 1.

Mit solchen Vorrichtungen ist es möglich eine entsprechende Verbindungsstange oder einen Längsträger - wie sie in der Wirbelsäulenchirurgie Verwendung finden - über ein Knochenfixationselement, vorzugsweise eine Knochenschraube mit dem Knochen zu verbinden um ein rigides Konstrukt zu erhalten.

Aus der US-A 4920959 WITZEL ET AL. ist ein *Fixateur externe* bekannt, der relativ umständlich ist. Insbesondere müssen die einzelnen Klammerelemente auf die Längsstäbe aufgefahren werden.

Aus der CH-A 632658 ist ein Implantat zur Fixierung von Knochen bekannt, bei welchem die Qualität der Arretierung direkt von der realisierbaren Vorspannkraft zwischen Bridenkörper und Knochen abhängig ist. Auch hier müssen - im Falle einer Verwendung des Implantats als Klammer - die einzelnen Klammerelemente auf zwei parallele Längsstäbe aufgefahren werden. Ein seitliches Aufsetzen ist somit nicht möglich. Dies kann bei einer Freihandanwendung, im speziellen perkutan und minimalinvasiv, schwer realisierbar sein und einen grossen Nachteil darstellen.

Aus der US 5290288, der WO 94/01049 A1 sowie der FR 2775587 A1 sind anderseits auch offene Klammern bekannt, welche allerdings alle den Nachteil besitzen, dass sie zu Ihrer Fixation eines zusätzlichen Elementes bedürfen. Die Klammerhalterung, d.h. die Knochenschraube muss zuerst im Knochen fixiert werden und kann nicht frei wählbar perkutan eingebracht werden, um dann erst die Klammer darauf mit der Knochenschraube positionieren und fixieren zu können. Aus diesem Grunde kann auch der Längsträger erst nach erfolgtem Positionieren der Klammer auf der Knochenschraube eingebracht werden.

Aus der WO95/13754 ist schliesslich eine Klammer bekannt, bei welcher ein Längsstab in den offenen Kanal einer Klammer seitlich einführbar ist und dort mittels einer durch die Klammer hindurchführbaren Feststellschraube festklemmbar ist. Die Feststellschraube hat aber nur diese eine Funktion und ist nicht als Knochenschraube ausgebildet, welche das ganze Konstrukt am Knochen befestigen könnte. Das gleiche gilt auch für die aus der DE-A 195 34 136 bekannte Halterung.

WO-A-2004/021901 offenbart eine Fixationsvorrichtung gemäss dem Oberbegriff des ersten Anspruchs.

Die aus dem Stand der Technik bekannten Vorrichtungen sind somit ganz allgemein kompliziert.

Hier will die Erfindung Abhilfe schaffen. Der Erfindung liegt die Aufgabe zugrunde, eine Vorrichtung zu schaffen, welche sehr einfach aufgebaut ist, möglichst flexibel anwendbar ist, sowie eine minimal invasive Operationstechnik erlaubt.

Die Erfindung löst die gestellte Aufgabe mit einer Vorrichtung, welche die Merkmale des Anspruchs 1 aufweist.

Die Vorteile der erfindungsgemässen Vorrichtung sind vielfältig und können wie folgt beschrieben werden:
- die Vorrichtung ist einteilig;
- der Längsträger kann vorausgehend in die Vorrichtung eingebracht und vorgeformt werden, während die Knochenschrauben erst gesetzt werden müssen, wenn deren Position genau bekannt ist;
- die Vorrichtung kann sowohl an einem Fixateur extern als auch an einem Fixateur interne eingesetzt werden;
- muss der Längsträger mittels einer weiteren Vorrichtung an der Wirbelsäule fixiert werden, kann eine solche weitere Vorrichtung, ohne diese mühsam über die Länge des Längsträgers zu schieben, einfach auf den Längsträger aufgesetzt werden; und
- der Längsträger kann vor dem Setzen der Knochenfixationselemente in den menschlichen Körper eingebracht werden, so dass die Positionen der nachher an den Wirbelkörpern zu befestigenden Knochenfixationselemente für den Operateur einfach zu bestimmen sind.

Die erfindungsgemässe Vorrichtung erlaubt die Aufnahme eines der Aussparung entsprechenden Längsträgers und einer Standard-Kopfverriegelungsschraube. Beim Festziehen der Kopf-Verriegelungsschraube wird der Längsträger in der erfindungsgemässen Vorrichtung blockiert.

Weitere vorteilhafte Ausgestaltungen der Erfindung sind in den abhängigen Ansprüchen gekennzeichnet.

In einer bevorzugten Ausführungsform wird das elastische Element durch mindestens zwei von der Bohrungswand quer zur Kanalachse in den Körper eindringende Schlitze teilweise vom Körper abgetrennt. Die Vorteile dieser Ausgestaltung sind im wesentlichen darin zusehen, dass das elastische Element mit dem Körper einstückig ausgebildet sein kann, so dass keine Teile verloren gehen können und eine einfache Herstellung der Vorrichtung ermöglicht wird.

In einer weiteren Ausführungsform ist das elastische Element in einer zur Kanalachse orthogonalen Querschnittsfläche als hakenförmiges Segment des Körpers ausgebildet und weist ein freies Ende auf. Der Vorteil dieser Ausführung liegt darin, dass die hakenförmige Ausbildung des elastischen Elementes eine rigide Fixierung des Längsträgers im Kanal ermöglicht.

In einer weiteren Ausführungsform wird der Kanal durch das freie Ende des elastischen Elementes verengt, so dass ein Längsträger in den Kanal einschnappbar ist. Der Vorteil dieser Ausgestaltung liegt im wesentlichen darin, dass der Längsträger auch im Kanal gehalten wird, wenn er nicht durch Anziehen des Knochenfixationselementes im Kanal fixiert ist.

In einer weiteren Ausführungsform ist die Bohrung mindestens teilweise konisch ausgebildet. Die Vorteile dieser Ausgestaltung sind im wesentlichen darin zusehen, dass das elastische Element mittels einer Konusverbindung zwischen der Bohrung und dem Knochenfixationselement auf einfache Weise in den Bereich des Kanals drückbar ist, so dass zur Fixierung des Längsträgers im Kanal keine weiteren Teile benötigt werden.

In einer weiteren Ausführungsform weist die Bohrung ein Innengewinde auf. Die Vorteile dieser Ausführung liegen darin, dass durch eine Gewindeverbindung zwischen der Bohrung und dem Knochenfixationselement eine rigide Verbindung zwischen dem Körper und dem Knochenfixationselement erreichbar ist.

In einer weiteren Ausführungsform weist die Bohrung mindestens teilweise eine sphärisch konkave Bohrungswand auf. Ferner ist ein komplementär sphärisches, radial elastisch deformierbares Spannelement mit einer zur Aufnahme des Knochenfixationselementes geeigneten Zentralbohrung in der Kavität schwenkbar gelagert und lösbar blockierbar. Die Vorteile dieser Ausgestaltung sind im wesentlichen darin zusehen, dass das Knochenfixationselement vor der Blockierung der Vorrichtung in der Bohrung polyaxial schwenkbar gelagert ist, so dass eine Fixierung des Knochenfixationselementes relativ zum Körper unter unterschiedlichen Winkeln möglich ist.

In einer weiteren Ausführungsform weist die Bohrung zwei konische Längenabschnitte auf, wobei sich je ein konischer Längenabschnitt gegen je eine Mündung der Bohrung erweitert. Die Vorteile dieser Ausgestaltung liegen darin, dass die Vorrichtung entweder mit einem nach posterior offenen Kanal implantiert werden kann, so dass der Längsträger nach dem Setzen der Knochenfixationsmittel in die Vorrichtung eingelegt werden kann, oder sie kann mit einem nach anterior offenen Kanal implantiert werden, so dass zuerst der Längsträger in den menschlichen Körper eingebracht werden kann, und nachher die notwendige Anzahl Vorrichtungen und Knochenfixationselemente implantiert und fixiert werden kann.

In einer weiteren Ausführungsform weist der Kanal in einem zur Kanalachse orthogonalen Querschnitt betrachtet eine polygonale Querschnittsfläche auf. Die Vorteile dieser Ausführung liegen darin, dass der Längsträger rotationsstabil mit der Vorrichtung verbindbar ist.

Nachstehend werden kurz zwei verschiedene Operationsmethoden für die erfindungsgemässe Vorrichtung beschrieben.

### Operationsmethode A:

A) Der Längsträger wird zuerst durch eine Stichinzision in den zu behandelnden Bereich des Patienten eingebracht und perkutan in die gewünschte Position geschoben;
B) Der Chirurg kann nun die Kontur des Längsträgers kontrollieren und einfach korrigieren;
C) Ist die Kontur des Längsträgers in Ordnung, kann die gewünschte Anzahl Vorrichtungen (Klammern) durch entsprechende Stichinzisionen perkutan eingebracht werden und direkt seitlich auf den Stab aufgebracht werden;
D) Anschliessend erfolgt das Bohren der Löcher für die Kopfverriegelungsschrauben;
E) Nun werden winkelstabile Kopfverriegelungsschrauben in die Bohrungen der Klammern eingedreht, aber noch nicht festgezogen.
F) Ist die Reposition in Ordnung, können die winkelstabilen Kopfverriegelungsschrauben definitiv festgezogen werden, so dass das aus dem Längsträger, den Klammern und den Knochenschrauben gebildete Konstrukt rigide wird und die Fraktur fixiert.

### Operationsmethode B:

1. Die erfindungsgemässe Vorrichtung (Klammer) wird mit posterior offenem Kanal relativ zum gewünschten Knochenteil positioniert;
2. Eine Kopfverriegelungsschraube wird durch die konische Bohrung der Klammer auf eine vorbestimmte Eindrehtiefe eingedreht, so dass eine Vorfixierung der Klammer erreicht wird;
3. Der Längsträger wird entsprechend den anatomischen Bedürfnissen angeformt;
4. Der Längsträger wird in den offenen Kanal der bereits vormontierten Klammer eingelegt;
5. Die Kopfverriegelungsschraube wird nun in der Klammer vollständig festgezogen, so dass eine Fixierung des Längsträgers an der Klammer erfolgt;
6. Die Fraktur wird mit einem geeigneten Repositionsinstrument über den Längsträger reponiert;
7. Die reponierten Knochenfragmente werden mittels der Klammern fixiert;
8. Wahlweise Ergänzung des Knochenfixations-Konstruktes mit Klammern.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 eine Explosionsdarstellung einer Knochenfixationsvorrichtung mit einem Längsträger, einer Ausführungsform der erfindungsgemässen Vorrichtung (Klammer) und einer Knochenschraube;
Fig. 2 einen Schnitt durch die in Fig. 1 dargestellte Ausführungsform der erfindungsgemässen Vorrichtung mit einer von der ersten Oberfläche her eingeführten Knochenschraube und einem Längsträger;
Fig. 3 einen Schnitt durch die in Fig. 1 dargestellte Ausführungsform der erfindungsgemässen Vorrichtung mit einer von der zweiten Oberfläche her eingeführten Knochenschraube und einem Längsträger;
Fig. 4 einen Schnitt durch eine weitere Ausführungsform der erfindungsgemässen Vorrichtung; und
Fig. 5 eine Aufsicht auf die in Fig. 4 dargestellte Ausführungsform der erfindungsgemässen Vorrichtung.

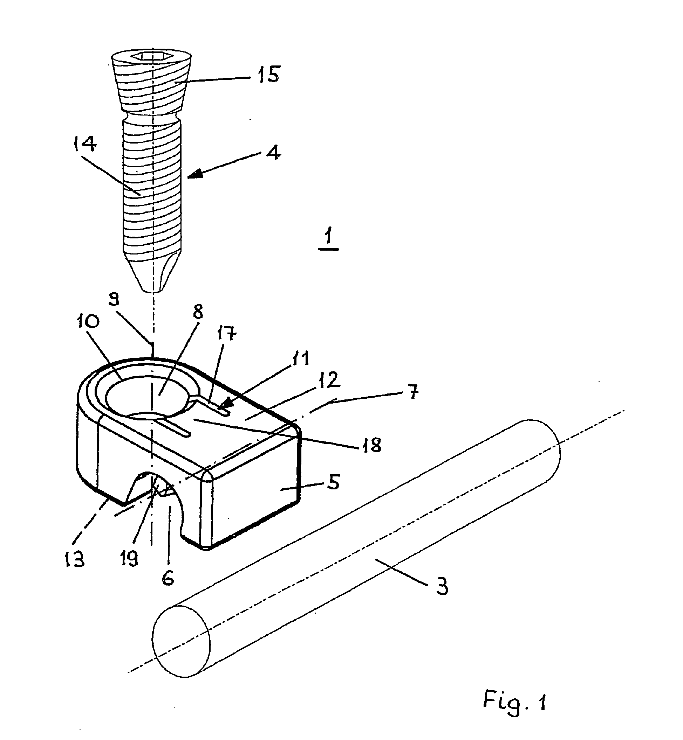

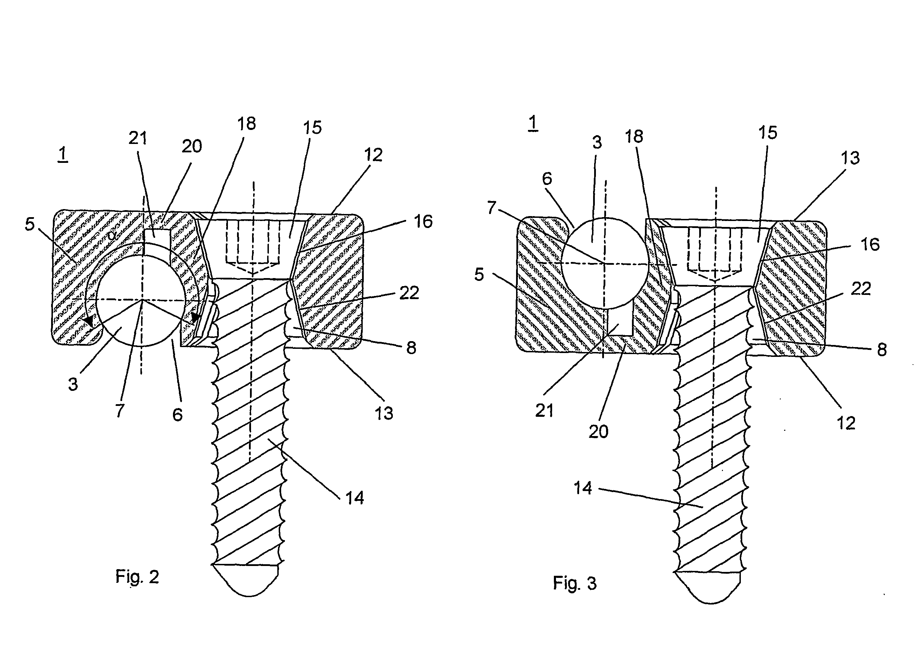

In den Fig. 1 ist eine Ausführungsform der Vorrichtung 1 zusammen mit einem als Knochenschraube ausgebildeten Knochenfixationselement 4 und einem Längsträger 3 dargestellt, wobei die Vorrichtung 1 im wesentlichen einen dreidimensionalen Körper 5 mit einer ersten Oberfläche 12 und parallel dazu einer zweiten Oberfläche umfasst. Der dreidimensionale Körper 5 dient als Verbindungselement zwischen Längsträger 3 und Knochenfixationselement 4 und umfasst eine den dreidimensionalen Körper 5 von der ersten Oberfläche 12 zur zweiten Oberfläche 13 durchdringende Bohrung 8 mit einer Bohrungsachse 9, welche zur Aufnahme des Knochenfixationselementes 4 geeignet ist. Ferner wird der dreidimensionale Körper 5 von einem gegen die zweite Oberfläche 13 offenen Kanal 6 mit einer senkrecht zur Bohrungsachse 9 stehenden Kanalachse 7 durchdrungen, so dass ein Längsträger 3 quer zum Knochenfixationselement 4 in den Kanal 6 einführbar ist. Die Bohrung 8 und der Kanal 6 sind derart angeordnet, dass sie sich nicht schneiden. Ferner umfasst der dreidimensionale Körper 5 ein durch zwei von der Bohrung 8 senkrecht zur Kanalachse 7 in den Körper 5 eindringenden Schlitzen 17 sowie durch die Bohrung 8 und den Kanal 6 begrenztes, hakenförmiges Segment 18, welches wegen der von der ersten zur zweiten Oberfläche 12;13 durchgehenden Schlitze 17 elastisch deformierbar ist und das elastische Element 11 zur Fixierung des Längsträgers 3 im Kanal 6 bildet. Im Bereich des hakenförmigen Segmentes 18 umschliesst der Kanal 6 den Längsträger 3 mit einem Umschlingungswinkel α > 180° (Fig. 2). Beim Eindrehen des Schraubenschaftes 14 des als Knochenschraube ausgebildeten Knochenfixationselementes 4 in einen Knochen wird das hakenförmige Segment 18 durch den konischen Kopf 15 des Knochenfixationselementes 4 derart deformiert, dass das freie Ende 19 des hakenförmigen Segmentes 18 gegen den in den Kanal 6 eingeführten Längsträger 3 gepresst wird und dieser im Kanal 6 rotativ und longitudinal fixiert wird.

Wie aus den Fig. 2 und 3 ersichtlich ist, weist der Kanal 6 eine den Körper 5 parallel zur Kanalachse 7 durchdringende Kerbe 21 auf, so dass zwischen dem Kerbgrund und der ersten Oberfläche 12 ein elastisch deformierbarer, das feste Ende 20 des hakenförmigen Segmentes 18 bildender Steg bleibt. Die Bohrung 8 weist zwei konische Längenabschnitt auf, welche sich jeweils sich zur ersten Oberfläche 12, respektive zur zweiten Oberfläche 13 hin erweitern. Die Bohrung 8 weist auf jedem der konischen Längenabschnitte ein konisches Innengewinde 22 auf, welches mit dem konischen Aussengewinde 16 am konischen Kopf 15 in Eingriff bringbar ist, so dass die Knochenschraube 14 im dreidimensionalen Körper 5 rigide fixierbar ist. Aufgrund der zwei konischen Längenabschnitte der Bohrung 8 besteht die Möglichkeit das Knochenfixationselement 4 je nach Anwendung von der ersten oder von der zweiten Oberfläche 12;13 her in den dreidimensionalen Körper 5 einzuführen. Damit ist auch die Möglichkeit gegeben, den dreidimensionalen Körper 5 so zu implantieren, dass der Längsträger 3 von der gegen den Kopf 15 gerichteten Seite (Fig. 3) oder von der dem Kopf 15 abgewandten Seite (Fig. 2) des dreidimensionalen Körpers 5 in den Kanal 6 einführbar ist.

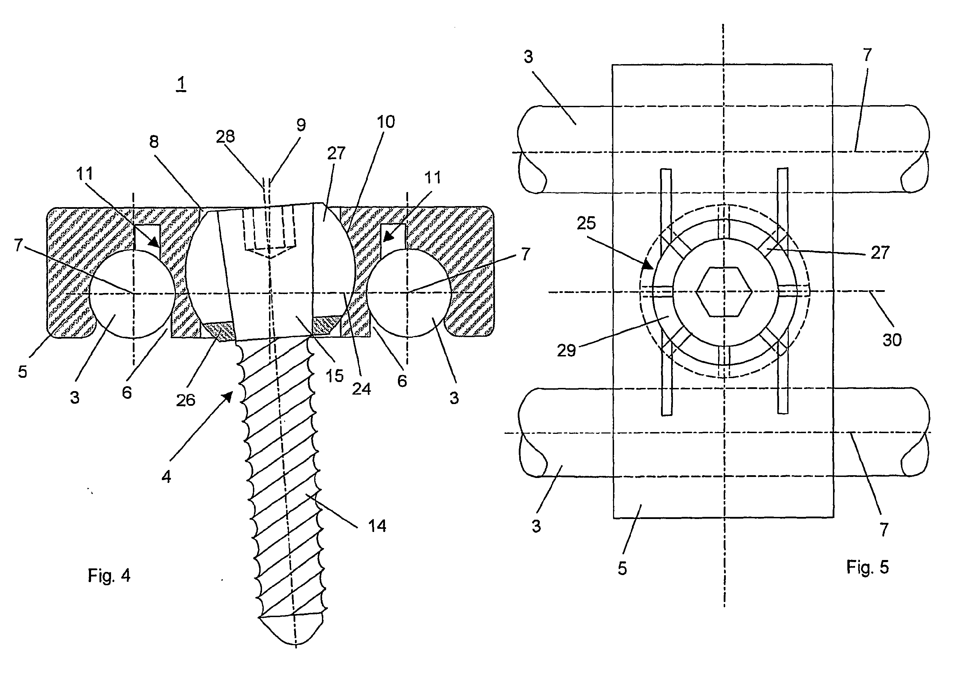

Die in den Fig. 4 und 5 dargestellte Vorrichtung 1 unterscheidet sich von der in den Fig. 1 bis 3 dargestellten Ausführungsform darin, dass
a) sie zwei Kanäle 6 umfasst, deren Kanalachsen 7 parallel zueinander verlaufen. Zwischen den Kanalachsen 7 angeordnet ist eine Symmetrieebene 30, welche senkrecht zu einer die Kanalachsen 7 orthogonal schneidenden Verbindungsgeraden 24 steht und diese Verbindungsgerade 24 halbiert. Die Ausgestaltung des zweiten Kanals 6 ist bezüglich der Symmetrieebene 30 spiegelsymmetrisch. Ebenfalls spiegelsymmetrisch zur Symmetrieebene 30 ausgebildet sind die zwei elastischen Elemente 11;
b) die Bohrungswand 10 der Bohrung 7 sphärisch konkav ausgebildet ist. Der konische Kopf 15 des als Knochenschraube 14 ausgebildeten Knochenfixationselementes 4 verjüngt sich gegen den Schraubenschaft 14 und ist in die komplementär konische Zentralbohrung 26 eines in der Bohrung 7 gelagerten, radial elastisch deformierbaren Spannelementes 25 eingeführt, so dass beim Anziehen des als Knochenschraube ausgebildeten Knochenfixationselementes 4 das elastische Spannelement 25 expandiert wird, wodurch in der Folge die elastischen Elemente 11 gegen die in die Kanäle 6 eingeführten Längsträger 3 gepresst werden und die Vorrichtung blockiert wird. Die elastische Deformierbarkeit des Spannelementes 25 wird durch acht Einkerbungen 27 erreicht, welche parallel zur Längsachse 28 der Zentralbohrung 26 und auf einem Teil deren Länge in die Wand 29 des Spannelementes 25 eindringen. Diese Ausgestaltung mit einer mindestens teilweise sphärischen Bohrungswand 10 und dem komplementären Spannelement 25 gestattet eine Schwenkung des Knochenfixationselementes 4 relativ zum dreidimensionalen Körper 5, so dass die Bohrungsachse 9 und die Längsachse 28 der Zentralbohrung 26 im Spannelement 25 einen Winkel zueinander einschliessen können.

## Patentansprüche

1. Vorrichtung (1) zur gegenseitigen Fixierung eines longitudinalen Längsträgers (3) mit einem Knochenfixationselement (4), wobei die Vorrichtung aus einem dreidimensionalen Körper (5) besteht, der einen offenen Kanal (6) mit der Kanalachse (7) zur Aufnahme des Längsträgers (3) sowie eine quer zur Kanalachse (7) verlaufende, den Körper (5) vollständig durchbohrenden Bohrung (8) mit einer Bohrungswand (10) besitzt, und wobei
mindestens ein Teil der zwischen dem Kanal (6) und der Bohrung (8) liegenden Bohrungswand (10) als elastisches Element (11) ausgebildet ist, **dadurch gekennzeichnet, dass** das elastische Element (11) durch Einführung eines Knochenfixationselementes (4) in die Bohrung (8) in den Bereich des Kanals (6) drückbar ist, so dass ein sich im Kanal befindlicher Längsträger (3) durch das elastische Element (11) rotativ und logitudinal festklemmbar ist

2. Vorrichtung (1) nach Anspruch 1, **dadurch gekennzeichnet, dass** das elastische Element (11) durch mindestens zwei von der Bohrungswand (10) quer zur Kanalachse (7) in den Körper (5) eindringende Schlitze (17) teilweise vom Körper (5) abgetrennt wird.

3. Vorrichtung (1) nach Anspruch 1 oder 2, **dadurch gekennzeichnet, dass** das elastische Element (11) in einer zur Kanalachse (7) orthogonalen Querschnittsfläche als hakenförmiges Segment (18) des Körpers (5) ausgebildet ist und ein freies Ende (19) aufweist.

4. Vorrichtung (1) nach Anspruch 3, **dadurch gekennzeichnet, dass** der Kanal (6) durch das freie Ende (19) des elastischen Elementes (11) verengt wird, so dass ein Längsträger (3) in den Kanal (6) einschnappbar ist.

5. Vorrichtung (1) nach einem der Ansprüche 1 bis 4, **dadurch gekennzeichnet, dass** die Bohrung (8) mindestens teilweise konisch ausgebildet ist.

6. Vorrichtung (1) nach einem der Ansprüche 1 bis 5, **dadurch gekennzeichnet, dass** die Bohrung (8) ein Innengewinde (22) aufweist.

7. Vorrichtung (1) nach einem der Ansprüche 1 bis 6, **dadurch gekennzeichnet, dass** sie zwei Kanäle (6) umfasst, welche auf gegenüberliegenden Seiten der Bohrung (8) angeordnet sind.

8. Vorrichtung (1) nach Anspruch 7, **dadurch gekennzeichnet, dass** sie zwei elastische Elemente (11) umfasst, wobei je ein Kanal (6) mindestens teilweise durch ein elastisches Element (11) begrenzt wird.

9. Vorrichtung (1) nach einem der Ansprüche 1 bis 8, **dadurch gekennzeichnet, dass** die Bohrung (8) mindestens teilweise eine sphärisch konkave Bohrungswand (10) aufweist, und dass ein komplementär sphärisches, radial elastisch deformierbares Spannelement (25) mit einer zur Aufnahme des Knochenfixationselementes (4) geeigneten Zentralbohrung (26) in der Kavität (24) schwenkbar gelagert und lösbar blockierbar ist.

10. Vorrichtung (1) nach einem der Ansprüche 1 bis 3, **dadurch gekennzeichnet, dass** die Bohrung (8) mindestens einen konischen Längenabschnitt mit einem Konuswinkel im Bereich von 5° und 25° aufweist.

11. Vorrichtung (1) nach Anspruch 4, **dadurch gekennzeichnet, dass** die Bohrung (8) mindestens einen konischen Längenabschnitt mit einem Konuswinkel im Bereich von 8° und 15° aufweist.

12. Vorrichtung (1) nach einem der Ansprüche 1 bis 5, **dadurch gekennzeichnet, dass** die Bohrung (8) zwei konische Längenabschnitte aufweist, wobei sich je ein konischer Längenabschnitt gegen je eine Mündung der Bohrung (8) erweitert.

13. Vorrichtung (1) nach einem der Ansprüche 1 bis 6, **dadurch gekennzeichnet, dass** sie zusätzlich ein Knochenfixationselement (4), vorzugsweise in Form einer Knochenschraube (14) aufweist, mit einer zur Einführung in den Knochen geeigneten Spitze (23), einem Gewindeschaft und einem zur Manipulation der Schraube geeigneten Kopf (15).

14. Vorrichtung nach Anspruch 7, **dadurch gekennzeichnet, dass** der Kopf (15) des Knochenfixationselementes (4) ein Aussengewinde (16) aufweist, welches vorzugsweise zum Innengewinde (22) korrespondiert.

15. Vorrichtung nach Anspruch 8, **dadurch gekennzeichnet, dass** die Steigung des Aussengewindes (16) zwischen 0,1 und 3,0 mm, vorzugsweise zwischen 0,25 und 1,50 mm liegt.

16. Vorrichtung nach einem der Ansprüche 6 bis 8, **dadurch gekennzeichnet, dass** der Kopf (15) des Knochenfixationselementes (4) sich gegen die Spitze (23) hin konisch verjüngt.

17. Vorrichtung nach Anspruch 9, **dadurch gekennzeichnet, dass** der sich konisch verjüngende Kopf (13) einen Konuswinkel im Bereich von 5° und 25°, vorzugsweise zwischen 8° und 15° aufweist.

18. Vorrichtung nach einem der Ansprüche 1 bis 17, **dadurch gekennzeichnet, dass** der Kanal (6) in einem zur Kanalachse (7) orthogonalen Querschnitt betrachtet eine polygonale Querschnittsfläche aufweist.

## Claims

1. Device (1) for the mutual fixing of a longitudinal carrier (3) with a bone fixation element (4), the device comprising a three-dimensional body (5), which has an open channel (6) with the channel axis (7) for accommodating the longitudinal carrier (3) as well as a borehole (8), which extends transversely to the axis (7) of the channel (6) completely through the body (5), the borehole having a wall (10), and wherein
at least a portion of the wall (10) of the borehole (8), lying between the channel (6) and the borehole (8), is constructed as an elastic element (11),
**characterized in that**
by introducing a bone fixation element (4) into the borehole (8) the elastic element (11) can be pressed into the region of the channel (6), so that a longitudinal carrier (3) in the channel can be clamped tightly rotationally and longitudinally by the elastic element (11 ).

2. The device (1) of claim 1, **characterized in that** the elastic element (11) is severed partly from the body (5) by at least two slots (17), which penetrate from the wall (10) of the borehole (8) transversely to the channel axis (7) into the body (5).

3. The device (1) of claims 1 or 2, **characterized in that** the elastic element (11) is constructed in a cross-sectional surface, orthogonal to the channel axes (7), as a hook-shaped segment (18) of the body (5) and has a free end (19).

4. The device (1) of claim 3, **characterized in that** the channel (6) is constricted by the free end (19) of the elastic element (11), so that a longitudinal carrier (3) can be snapped into the channel (6).

5. The device (1) of one of the claims 1 to 4, **characterized in that**, at least partly, the borehole (8) is constructed conically.

6. The device (1) of one of the claims 1 to 5, **characterized in that** the borehole (8) has an internal thread (22).

7. The device (1) of one of the claims 1 to 6, **characterized in that** it comprises two channels (6), which are disposed on opposite sides of the borehole (8).

8. The device (1) of claim 7, **characterized in that** it comprises two elastic elements (11), each channel (6) being bounded at least partly by an elastic element (11).

9. The device (1) of one of the claims 1 to 8, **characterized in that** the borehole (8) has at least partly a spherically concave borehole wall (10) and **in that** a complementary spherical tensioning element (25), elastically deformable in the radial direction, is supported pivotably in the cavity (24) with a central borehole (26), suitable for accommodating the bone fixation element (4) and can be blocked releasably.

10. The device of one of the claims 1 to 3, **characterized in that** the borehole (8) has at least one conical longitudinal section with a conical angle ranging from 5° to 25°.

11. The device (1) of claim 4, **characterized in that** the borehole (8) has at least one conical longitudinal section with a conical angle ranging from 8° to 15°.

12. The device (1) of one of the claims 1 to 5, **characterized in that** the borehole (8) has two conical longitudinal sections, each conical longitudinal section expanding in the direction of the mouth of the borehole (8).

13. The device (1) of one of the claims 1 to 6, **characterized in that** it additionally has a bone fixation element (4), preferably in the form of a bone screw (14), with a tip (23), suitable for introducing it into the bone, a threaded shaft and a head (15), suitable for manipulating the screw.

14. The device of claim 7, **characterized in that** the head (15) of the bone fixation element (4) has an external thread (16), which corresponds preferably to the internal thread (22).

15. The device of claim 8, **characterized in that** the pitch of the external thread (16) is between 0.1 and 3.0 mm in preferably between 0.25 and 1.50 mm.

16. The device of one of the claims 6 to 8, **characterized in that** the head (15) of the bone fixation element (4) tapers conically in the direction of the tip (23).

17. The device of claim 9, **characterized in that** the conically tapering head (13) has a conical angle ranging from 5° to 25° in preferably from 8° to 15°.

18. The device of one of the claims 1 to 17, **characterized in that** the channel (6), viewed in a cross-section orthogonal to the channel axis (7), has a polygonal cross-sectional surface.

## Revendications

1. Dispositif (1) pour la fixation mutuelle d'un élément de maintien longitudinal (3) avec un élément de fixation osseuse (4), le dispositif se composant d'un corps tridimensionnel (5) qui possède un canal ouvert (6) avec l'axe de canal (7) pour loger l'élément de maintien longitudinal (3) ainsi qu'un alésage (8) comportant une paroi d'alésage (10), traversant entièrement le corps (5) et s'étendant transversalement à l'axe de canal (7), et
au moins une partie de la paroi d'alésage (10) située entre le canal (6) et l'alésage (8) étant réalisée sous forme d'élément élastique (11), **caractérisé en ce que** l'élément élastique (11) peut être comprimé par l'insertion d'un élément de fixation osseuse (4) dans l'alésage (8) dans la zone du canal (6), de sorte qu'un élément de maintien longitudinal (3) se trouvant dans le canal peut être bloqué en rotation et longitudinalement par l'élément élastique (11).

2. Dispositif (1) selon la revendication 1, **caractérisé en ce que** l'élément élastique (11) est en partie séparé du corps (5) par au moins deux fentes (17) rentrant dans le corps (5) depuis la paroi d'alésage (10) transversalement à l'axe de canal (7).

3. Dispositif (1) selon la revendication 1 ou 2, **caractérisé en ce que** l'élément élastique (11) est réalisé, dans une aire de section transversale orthogonale à l'axe de canal (7), selon un segment du corps (5) en forme de crochet (18), et présente une extrémité libre (19).

4. Dispositif (1) selon la revendication 3, **caractérisé en ce que** le canal (6) est rétréci par l'extrémité libre (19) de l'élément élastique (11) de sorte qu'un élément de maintien longitudinal (3) peut être encliqueté dans le canal (6).

5. Dispositif (1) selon l'une des revendications 1 à 4, **caractérisé en ce que** l'alésage (8) est réalisé au moins en partie sous forme conique.

6. Dispositif (1) selon l'une des revendications 1 à 5, **caractérisé en ce que** l'alésage (8) présente un taraudage (22).

7. Dispositif (1) selon l'une des revendications 1 à 6, **caractérisé en ce qu'**il comprend deux canaux (6) qui sont disposés sur des côtés opposés de l'alésage (8).

8. Dispositif (1) selon la revendication 7, **caractérisé en ce qu'**il comprend deux éléments élastiques (11), chaque canal (6) étant délimité au moins en partie par un élément élastique (11).

9. Dispositif (1) selon l'une des revendications 1 à 8, **caractérisé en ce que** l'alésage (8) présente au moins en partie une paroi d'alésage sphérique concave (10), et **en ce qu'**un élément de serrage (25) ayant une forme sphérique complémentaire, déformable radialement de manière élastique et comportant un alésage central (26) approprié pour recevoir l'élément de fixation osseuse (4), est logé de manière à pouvoir pivoter dans la cavité (24) et peut y être bloqué de manière amovible.

10. Dispositif (1) selon l'une des revendications 1 à 3, **caractérisé en ce que** l'alésage (8) présente au moins une section longitudinale conique avec un angle de conicité dans une plage allant de 5° à 25°.

11. Dispositif (1) selon la revendication 4, **caractérisé en ce que** l'alésage (8) présente au moins une section longitudinale conique avec un angle de conicité dans une plage allant de 8° à 15°.

12. Dispositif (1) selon l'une des revendications 1 à 5, **caractérisé en ce que** l'alésage (8) présente deux sections longitudinales coniques, chaque section longitudinale conique s'élargissant vers une ouverture respective de l'alésage (8).

13. Dispositif (1) selon l'une des revendications 1 à 6, **caractérisé en ce qu'**il présente en plus un élément de fixation osseuse (4), de préférence sous la forme d'une vis à os (14), comprenant une pointe (23) appropriée à une insertion dans l'os, une tige filetée et une tête appropriée à la manipulation de la vis (15).

14. Dispositif selon la revendication 7, **caractérisé en ce que** la tête (15) de l'élément de fixation osseuse (4) présente un filetage extérieur (16) qui correspond de préférence au taraudage (22).

15. Dispositif selon la revendication 8, **caractérisé en ce que** le pas du filetage extérieur (16) est compris entre 0,1 et 3,0 mm, de préférence entre 0,25 et 1,50 mm.

16. Dispositif selon l'une des revendications 6 à 8, **caractérisé en ce que** la tête (15) de l'élément de fixation osseuse (4) se rétrécit de manière conique vers la pointe (23).

17. Dispositif selon la revendication 9, **caractérisé en ce que** la tête (15) se rétrécissant de manière conique présente un angle de conicité dans une plage allant de 5° à 25°, de préférence de 8° à 15°.

18. Dispositif selon l'une des revendications 1 à 17, **caractérisé en ce que** le canal (6), vu selon une coupe droite orthogonale à l'axe de canal (7), présente une aire de section transversale polygonale.
